# EUROPEAN PATENT APPLICATION

(11) **EP 3 649 929 A1**
(43) Date of publication of application: **13.05.2020**
(21) Application number: 18204535.1
(22) Date of filing: 06.11.2018
(51) Int. Cl.: A61B 5/022, A61B 5/00

(54) **AN APPARATUS FOR USE WITH A WEARABLE CUFF**

(71) Applicant: Koninklijke Philips N.V., 5656 AG Eindhoven (NL)
(72) Inventor: LAMICHHANE, Bishal, 5656 AE Eindhoven (NL); KUENEN, Maarten Petrus Joseph, 5656 AE Eindhoven (NL); MUEHLSTEFF, Jens, 5656 AE Eindhoven (NL); MONROE, Charles Christy, 5656 AE Eindhoven (NL); SCHMITT, Lars, 5656 AE Eindhoven (NL)
(74) Representative: de Haan, Poul Erik

(57) **Abstract**

There is provided an apparatus (12) for use with a wearable cuff (14). The wearable cuff (14) is for use in measuring blood pressure. The apparatus (12) is configured to acquire a plurality of pressure signals. Each of the plurality of pressure signals are indicative of a pressure in the wearable cuff (14) during a period for measuring blood pressure. The apparatus (12) is configured to, for each of the plurality of acquired pressure signals, determine a measure of a quality of the acquired pressure signal based on one or more parameters derived from artifacts identified in the acquired pressure signal. The apparatus (12) is also configured to determine a measure of a quality of the wearable cuff (14) based on the determined measures of the quality of the acquired pressure signals.

## Description

### BACKGROUND OF THE INVENTION

Blood pressure (BP) or, more precisely, arterial blood pressure, is the pressure exerted by circulating blood on the arterial vessel walls. It is one of the key vital signs to establish patient well-being and therefore needs to be monitored for patients at risk. Blood pressure is a periodic signal, which rises at each contraction of the heart and decreases in between heart beats. It is typically described by systolic blood pressure (SBP), diastolic blood pressure (DBP) and mean arterial blood pressure (MAP), where systolic blood pressure is the maximum blood pressure during heart cycle, diastolic blood pressure is the minimum blood pressure during heart cycle, and mean arterial blood pressure is the average blood pressure during a heart cycle.

Different techniques exist by which blood pressure can be determined and these can be classified as invasive or non-invasive blood pressure measurement techniques. Typically, non-invasive blood pressure (NIBP) measurement techniques are cuff-based, which require an inflatable cuff to be placed around a limb (which is usually the upper arm) of a subject. The pressure in the cuff is then changed to infer blood pressure. There are two common methods that use a cuff in this way, which are referred to in the art as the auscultatory method and the oscillometric method respectively.

The auscultatory method for NIBP measurement is based on the appearance and disappearance of sounds created by the artery under the cuff during the period that the cuff pressure is changed. These sounds are referred to in the art as Korotkoff sounds. The pressures at which the Korotkoff sounds appear and vanish are indicative of DBP and SBP with Korotkoff sounds appearing at each heart beat between DBP and SBP. The measurement of sound can be performed manually with a stethoscope that is placed over the artery just below the cuff, or in an automated way with a microphone under the cuff.

In the oscillometric method for NIBP measurement, the systolic and diastolic blood pressure values are based on small volume oscillations or pressure oscillations that are induced in the cuff by each heart beat. The amplitude of these volume or pressure oscillations depends on the difference between the cuff pressure and the actual arterial blood pressure. Systolic blood pressure and diastolic blood pressure are then determined as the cuff pressure where the volume or pressure oscillations have amplitudes of a certain fraction of the maximum oscillation amplitude. These fractions are typically heuristically determined.

In both the auscultatory and oscillometric method, the mean arterial pressure is typically calculated as: MAP = 2/3*DBP + 1/3*SBP.

The oscillometric and auscultatory measurement methods can be performed either during inflation of the cuff or during deflation of the cuff. Conventionally, measurements during deflation are used, in which the cuff is rapidly inflated to a level above the SBP where the blood flow in the artery under the cuff is blocked, after which cuff pressure is decreased gradually or in a stepwise manner. During deflation, the volume or pressure oscillations or the Korotkoff sounds are measured. While deflation stage NIBP measurement is well-established, an issue exists in the discomfort it introduces to the subject. In particular, the subject is exposed to a relatively high cuff pressure for a certain amount of time and pressures above a certain level can be uncomfortable and even painful, either due to the pressure exerted by the cuff itself or due to a build-up of venous blood in the clamped extremity (namely, venous pooling). The longer these pressures are applied to the subject, the higher the discomfort level is for the subject.

Another issue with utilizing the deflation based NIBP measurement is that the process of inflating the cuff and then deflating the cuff can be considerably long, where each NIBP measurement during deflation typically takes 40 seconds to complete. Also, since a defined maximum pressure level needs to be achieved before the deflation procedure can be initiated, the subject is exposed to a maximum cuff pressure that is higher than that required for the blood pressure measurement itself. Furthermore, the inherent variability of blood pressure over time can distort a single blood pressure measurement.

Due to these issues, apparatus have been developed that determine pressure oscillations during inflation of the cuff. These apparatus can reduce the discomfort as a blood pressure measurement may be accomplished in less time using the inflation stage, rather than the deflation stage. In order to have the measurement time as short as possible without sacrificing accuracy, the cuff inflation speed can be made pulse rate dependent such that the optimal number of pressure oscillations (i.e. enough to assure a certain accuracy but no more) is present for determining blood pressure. Thus, once the pressure range of interest is achieved at the inflation stage, pressure relief may be initiated so as to reduce the overall measurement time and the pressure-based discomfort.

However, in general, non-invasive blood pressure (NIBP) measurements are easily distorted due to the small amplitude of the pressure oscillations. One such distortion results from slippage of the cuff, which introduces an artifact in the pressure signal acquired from the cuff. The artifact can be misinterpreted as pressure oscillations in the pressure signal. As such, existing inflation-based techniques that measure blood pressure from this pressure signal can lead to inaccurate (e.g. false) or even unsuccessful blood pressure measurements. In the worst case, the inaccurate or unsuccessful blood measurements can result in a medical professional (e.g. a physician or nurse) making an incorrect diagnosis or initiating the wrong action. There exist methods that discard blood pressure measurements on detection of an artifact. However, it may be the case that the artifact does not actually originate from slippage of the cuff and instead is a result of the quality of the cuff degrading.

This can be problematic as, if a cuff with a low quality is not discarded timely, various consequences may result, e.g. inflation-based NIBP measurements may need to be repeated more often, there may be a high failure rate for inflation-based NIBP measurements, deflation-based NIBP measurements may be used instead (which are uncomfortable for the subject), and so on. US 7, 070, 566 discloses a technique for obtaining an overall quality assessment based on individual quality values for feature measurements of pulse oscillations. However, there may be confounding factors (e.g. cuff slippage) that affect the quality of pressure signals for a wearable cuff, which may cause an inaccurate and unreliable quality assessment. For example, the cuff may be of high quality when a low quality assessment is made due to the negative influence that these confounding factors have on the pulse.

### SUMMARY OF THE INVENTION

As noted above, the limitation associated with existing techniques for quality assessment of a wearable cuff is that they are limited in their ability to provide an accurate and reliable assessment of quality because of confounding factors (e.g. cuff slippage) on a single measurement instant, which can occur due to a particular measurement configuration in that instant. These confounding factors may affect the quality assessment and cause erroneous results. It would thus be valuable to address this limitation and provide an improved quality assessment.

Therefore, according to a first aspect, there is provided an apparatus for use with a wearable cuff. The wearable cuff is for use in measuring blood pressure. The apparatus is configured to acquire a plurality of pressure signals. Each of the plurality of pressure signals are indicative of a pressure in the wearable cuff during a period for measuring blood pressure. The apparatus is configured to, for each of the plurality of acquired pressure signals, determine a measure of a quality of the acquired pressure signal based on one or more parameters derived from artifacts identified in the acquired pressure signal. The apparatus is also configured to determine a measure of a quality of the wearable cuff based on the determined measures of the quality of the acquired pressure signals.

In some embodiments, the apparatus may be configured to determine the measure of the quality of the wearable cuff based on a trend in the determined measures of the quality of the acquired pressure signals. In some embodiments, the apparatus may be configured to determine the measure of the quality of the wearable cuff based on the determined measures of the quality of the acquired pressure signals and information on one or more properties of the wearable cuff.

In some embodiments, the apparatus may be configured to, for each of the plurality of acquired pressure signals, determine the measure of the quality of the acquired pressure signal by being configured to compare the one or more parameters to one or more corresponding parameters associated with at least one wearable cuff for which a measure of a quality of the at least one wearable cuff is predetermined.

In some embodiments, the measures of the quality of the acquired pressure signals may comprise binary measures of the quality of the acquired pressure signals or probabilistic measures of the quality of the acquired pressure signals.

In some embodiments, the determined measure of the quality of the wearable cuff may be indicative of a suitability of the wearable cuff for use in measuring blood pressure. In some embodiments, the determined measure of the quality of the wearable cuff may be indicative of the wearable cuff being suitable for use in measuring blood pressure during inflation of the wearable cuff, the wearable cuff being suitable for use in measuring blood pressure during deflation of the wearable cuff, or the wearable cuff being unsuitable for use in measuring blood pressure.

In some embodiments, the apparatus may be configured to determine whether the wearable cuff is to be discarded or replaced based on the determined measure of the quality of the wearable cuff.

In some embodiments, the one or more parameters may comprise any one or more of: a number of occurrences of artifacts in the acquired pressure signal, a number of occurrences of artifacts in the acquired pressure signal that fall within a cardiac cycle, an average time between occurrences of artifacts in the acquired pressure signal, an average intensity of artifacts in the acquired pressure signal, a ratio of a cumulative sum of amplitudes of artifacts in the acquired pressure signal to a cumulative sum of amplitudes of oscillations in the acquired pressure signal, a ratio of an average of amplitudes of artifacts in the acquired pressure signal to an average of amplitudes of oscillations in the acquired pressure signal, and a ratio of a maximum of amplitudes of artifacts in the acquired pressure signal to a maximum of amplitudes of oscillations in the acquired pressure signal.

In some embodiments, the period for measuring blood pressure may comprise a period in which the wearable cuff is inflated and/or deflated for measuring blood pressure.

In some embodiments, the apparatus may be configured to determine the measures of the quality of the acquired pressure signals using any one or more of a trained classifier, a regression analysis, a support vector machine, and a neural network algorithm.

In some embodiments, the apparatus may be configured to identify the wearable cuff for which the measure of the quality is determined.

In some embodiments, the apparatus may be configured to control a user interface to render an indication of the measure of the quality of the wearable cuff.

According to a second aspect, there is provided a method of operating an apparatus for use with a wearable cuff. The wearable cuff is for use in measuring blood pressure. The method comprises acquiring a plurality of pressure signals. Each of the plurality of pressure signals are indicative of a pressure in the wearable cuff during a period for measuring blood pressure. The method comprises, for each of the plurality of acquired pressure signals, determining a measure of a quality of the acquired pressure signal based on one or more parameters derived from artifacts identified in the acquired pressure signal. The method also comprises determining a measure of a quality of the wearable cuff based on the determined measures of the quality of the acquired pressure signals.

According to a third aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitation associated with existing techniques is addressed. In particular, the above-described aspects and embodiments enable an improved quality assessment for a wearable cuff by determining a measure of a quality of the wearable cuff in the manner described. In particular, as the overall assessment of the quality of the wearable cuff is made based on measures of a quality of a plurality of acquired pressure signal in respect of various periods for measuring blood pressure and using one or more parameters derived from artifacts identified in the plurality of acquired pressure signal, the quality assessment is more reliable since confounding factors (e.g. cuff slippage) on a single measurement instant, which can occur due to a particular measurement configuration in that instant, can be detected and accounted for in the measures of the quality of the acquired pressure signals that are determined. For example, the above-described aspects and embodiments make it possible to identify situations where confounding factors (e.g. cuff slippage) are affecting the quality of pressure signals for a wearable cuff, while the wearable cuff itself is actually of a high quality, and can thus provide a more reliable quality assessment for the wearable cuff.

In this way, a more reliable inference about cuff quality degradation can be obtained, such that a wearable cuff with low quality can be discarded timely. The improved quality assessment for a wearable cuff can aid in increasing the reliability of blood pressure measurements in which a wearable cuff is used, which in turn reduces or even eliminates failed blood pressure measurements. The blood pressure measurements can also be acquired faster, since the need to repeat blood pressure measurements is reduced or even eliminated.

The limitation associated with existing techniques is therefore addressed by way of the above-described aspects and embodiments.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a simplified schematic illustration of an apparatus in use with a wearable cuff according to an example embodiment;
Fig. 2 is a flow chart illustrating a method of operating the apparatus according to an embodiment;
Figs. 3(a)-(b) are graphical illustrations of a pressure signal over time for two different wearable cuffs according to an embodiment;
Figs. 3(c)-(d) are graphical illustrations of oscillations of a rate signal over time for the two different wearable cuffs according to the embodiment; and
Figs. 4(a)-(f) are graphical illustrations of example parameters derived from artifacts identified in an acquired pressure signal from two different cuffs according to the embodiment.

### DETAILED DESCRIPTION OF EMBODIMENTS

There is provided herein an apparatus for use with a wearable cuff (or clamp unit), which overcomes the limitation associated with existing techniques. The apparatus described herein can be a non-invasive blood pressure (NIBP) apparatus (e.g. a NIBP measurement apparatus), a server (e.g. a decision server), or any other type of apparatus.

The wearable cuff referred to herein is for use in measuring blood pressure. The wearable cuff is inflatable to pressurize a measurement site of a subject (e.g. a patient). In this way, the wearable cuff can pressurize an artery in the measurement site of the subject. The wearable cuff referred to herein can also be inflatable to pressurize a phantom (or dummy) of a body part, e.g. an arm or leg phantom. The phantom may, for example, comprise a rigid cylinder. Typically, the wearable cuff can be supplied with a fluid (e.g. a gas, such as air, or any other fluid) suitable for inflating the wearable cuff. The wearable cuff can be inflatable to pressurize the measurement site of a subject (and thus an artery in the measurement site of the subject) or a phantom of a body part at the pressure of the fluid in the wearable cuff. The wearable cuff can be for use in NIBP measurements, such as deflation-based NIBP measurements and/or inflation-based NIBP measurements.

The wearable cuff is configured to be worn on or around (e.g. wrapped around, attached to, or fastened to) the measurement site of the subject and/or a phantom of a body part. The measurement site of the subject can be any site on the body of the subject that is suitable for use in measuring a blood pressure of the subject, such as any site on the body of the subject that comprises an artery. For example, the measurement site of the subject may be located on a limb of the subject, such as an arm (e.g. an upper arm or a forearm) of the subject. Thus, the wearable cuff can be configured to be worn on or around (e.g. wrapped around, attached to, or fastened to) a limb of the subject. **The subject referred to herein can be, for example, an adult or a pediatric subject, e.g. an infant, a child or an adolescent.** An infant can, for example, be a neonate, such as a pre-term or premature infant, a full-term infant or a post-term infant.

Briefly, the apparatus described herein is configured to acquire a plurality of pressure signals. Each of the plurality of pressure signals is indicative of a pressure in the wearable cuff during a period for measuring blood pressure. That is, each of the plurality of acquired pressure signals are indicative of a pressure in the wearable cuff during a respective period of a plurality of periods for measuring blood pressure. The apparatus described herein is also configured to, for each of the plurality of acquired pressure signals, determine a measure of a quality of the acquired pressure signal based on one or more parameters derived from artifacts identified (or detected) in the acquired pressure signal. The apparatus described herein is further configured to determine a measure of a quality of the wearable cuff based on the determined measures of the quality of the acquired pressure signals.

Any references herein to "pressure signal" or "acquired pressure signal" will be understood to mean a pressure signal or an acquired pressure signal indicative of the pressure in the wearable cuff during a period for measuring blood pressure. Similarly, any references herein to "acquired plurality of pressure signals" will be understood to mean an acquired plurality of pressure signals each being indicative of the pressure in the wearable cuff during a period for measuring blood pressure. A pressure signal or an acquired pressure signal indicative of a pressure in the wearable cuff during a blood pressure measurement may include oscillations in pressure (or pressure oscillations).

The apparatus described herein can be implemented in numerous ways, with software and/or hardware, to perform the various functions described herein. In particular implementations, the apparatus can comprise a plurality of software and/or hardware modules, each configured to perform, or that are for performing, individual or multiple steps of the method described herein. The apparatus may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The apparatus may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

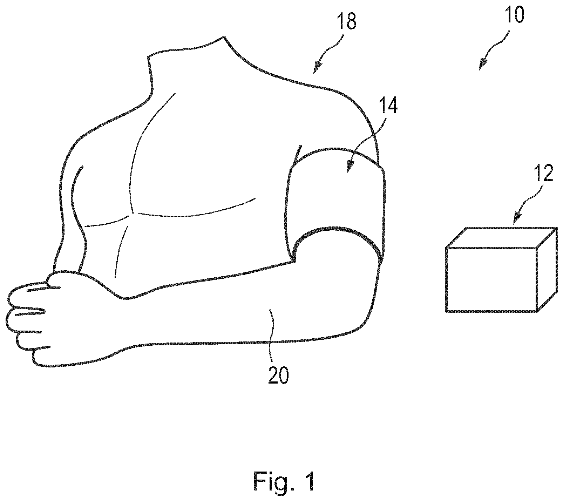

Fig. 1 illustrates the apparatus 12 according to an example embodiment in use with a wearable cuff 14. The wearable cuff 14 is for use in measuring blood pressure. There is thus also provided a system 10 comprising the apparatus 12 and the wearable cuff 14. As mentioned earlier, the wearable cuff 14 is inflatable to pressurize a measurement site 20 of a subject 18. In the example embodiment illustrated in Fig. 1, the measurement site 20 of the subject 18 is located on the arm or, more specifically, the upper arm of the subject 18. Thus, the cuff 14 is worn on or around (e.g. wrapped around, attached to, or fastened to) the upper arm of the subject 18 in this illustrated example embodiment.

Although not illustrated in Fig. 1, in some embodiments, the system 10 may comprise at least one pressure sensor. The at least one pressure sensor can be configured to obtain one or more pressure signals. In these embodiments, the apparatus 12 can be configured to acquire one or more of the plurality of pressure signals from the at least one pressure sensor. Therefore, the acquired plurality of pressure signals may comprise at least one current pressure signal (e.g. at least one pressure signal currently or presently obtained) according to some embodiments.

In some embodiments, the apparatus 12 described herein may comprise at least one pressure sensor. Alternatively or in addition, at least one pressure sensor may be external to (e.g. separate to or remote from) the apparatus 12. For example, in some embodiments, the cuff 14 itself may comprise at least one pressure sensor. In some embodiments, the at least one pressure sensor can be controllable by the apparatus 12 or, more specifically, a processor (not illustrated in Fig. 1) of the apparatus 12 to obtain the plurality of pressure signals. The apparatus 12 (or a processor of the apparatus 12) may communicate with and/or connect to the at least one pressure sensor in any suitable way to control the at least one pressure sensor.

Although also not illustrated in Fig. 1, in some embodiments, the system 10 may comprise a communications interface (or communications circuitry). In some embodiments, the apparatus 12 described herein may comprise a communications interface. Alternatively or in addition, the communications interface may be external to (e.g. separate to or remote from) the apparatus 12. The communications interface can be for enabling the apparatus 12, or components of the apparatus 12 (e.g. a processor of the apparatus 12), to communicate with and/or connect to one or more other components, sensors, interfaces, devices, or memories (such as any of those described herein). For example, the communications interface can be for enabling the apparatus 12 (or a processor of the apparatus 12) to communicate with and/or connect to the at least one pressure sensor described earlier.

The communications interface may enable the apparatus 12, or components of the apparatus 12, to communicate and/or connect in any suitable way. For example, the communications interface may enable the apparatus 12, or components of the apparatus 12, to communicate and/or connect wirelessly, via a wired connection, or via any other communication (or data transfer) mechanism. In some wireless embodiments, for example, the communications interface may enable the apparatus 12, or components of the apparatus 12, to use radio frequency (RF), Bluetooth, or any other wireless communication technology to communicate and/or connect.

Although also not illustrated in Fig. 1, in some embodiments, the system 10 may comprise at least one memory. In some embodiments, the apparatus 12 described herein may comprise at least one memory. Alternatively or in addition, at least one memory may be external to (e.g. separate to or remote from) the apparatus 12. For example, a hospital database may comprise the memory, the memory may be a cloud computing resource, or similar. The apparatus 12 (or a processor of the apparatus 12) may be configured to communicate with and/or connect to at least one memory. A memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory can be configured to store program code that can be executed by a processor (such as a processor of the apparatus 12) to cause the apparatus 12 to operate in the manner described herein.

Alternatively or in addition, in some embodiments, at least one memory can be configured to store information required by or resulting from the method described herein. For example, in some embodiments, at least one memory may be configured to store any one or more of: one or more pressure signals (e.g. obtained by at least one pressure sensor), one or more of the plurality of pressure signals acquired by the apparatus 12, one or more of the determined measures of the quality of the acquired pressure signals (or an indication of one or more of the determined measures of the quality of the acquired pressure signals), the determined measure of the quality of the wearable cuff (or an indication of the determined measure of the quality of the wearable cuff), or any other information, or any combination of information, required by or resulting from the method described herein. In some embodiments, the apparatus 12 (or a processor of the apparatus 12) can be configured to control at least one memory to store information required by or resulting from the method described herein.

In embodiments where at least one memory is configured to store one or more pressure signals (e.g. obtained by at least one pressure sensor), the apparatus 12 can be configured to access at least one memory to acquire one or more of the plurality of pressure signals. Thus, in some embodiments, a plurality of pressure signals may be pooled together for storage in at least one memory. Therefore, the acquired plurality of pressure signals may comprise at least one historical pressure signal (e.g. at least one pressure signal obtained in the past or at an earlier time) according to some embodiments. In some embodiments, the acquired plurality of pressure signals may comprise at least one current pressure signal and at least one historical pressure signal.

Although also not illustrated in Fig. 1, the system 10 may comprise a user interface. In some embodiments, the apparatus 12 described herein may comprise the user interface. Alternatively or in addition, the user interface may be external to (e.g. separate to or remote from) the apparatus 12. The apparatus 12 (or a processor of the apparatus 12) may be configured to communicate with and/or connect to a user interface. In some embodiments, the apparatus 12 (or a processor of the apparatus 12) can be configured to control the user interface to operate in the manner described herein.

The user interface can be configured to render (or output, display, or provide) information required by or resulting from the method described herein. For example, in some embodiments, the user interface may be configured to render (or output, display, or provide) one or more pressure signals (e.g. obtained by at least one pressure sensor), one or more of the plurality of pressure signals acquired by the apparatus 12, one or more of the determined measures of the quality of the acquired pressure signals (or an indication of one or more of the determined measures of the quality of the acquired pressure signals), the determined measure of the quality of the wearable cuff (or an indication of the determined measure of the quality of the wearable cuff), or any other information, or any combination of information, required by or resulting from the method described herein. Alternatively or in addition, the user interface can be configured to receive a user input. For example, the user interface may allow a user to manually enter information or instructions, interact with and/or control the apparatus 12. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

Although also not illustrated in Fig 1, the apparatus 12 may comprise a battery or other power supply for powering the apparatus 12 or means for connecting the apparatus 12 to a mains power supply. It will also be understood that the apparatus 12 may comprise any other component to those described herein or any combination of components.

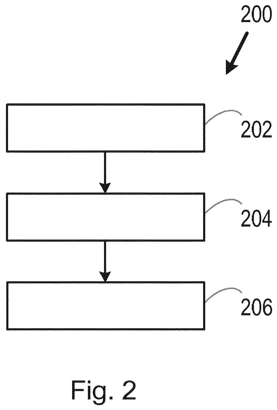

Fig. 2 illustrates a method 200 of operating the apparatus 12 described herein for use with a wearable cuff 14 according to an embodiment. As mentioned earlier, the wearable cuff 14 is for use in measuring blood pressure. The method 200 can generally be performed by or under the control of the apparatus 12 (or a processor of the apparatus 12) described earlier.

At block 202 of Fig. 2, a plurality of pressure signals are acquired. More specifically, the apparatus 12 acquires the plurality of pressure signals. As mentioned earlier, in some embodiments, the apparatus 12 may be configured to acquire one or more of the plurality of pressure signals from at least one pressure sensor. Alternatively or in addition, the apparatus 12 may be configured to acquire one or more of the plurality of pressure signals from at least one memory. Thus, as mentioned earlier, the acquired plurality of pressure signals may comprise at least one current pressure signal and at least one historical pressure signal according to some embodiments.

Each of the plurality of pressure signals are indicative of a pressure in the wearable cuff 14 during a period for measuring blood pressure. That is, each of the plurality of acquired pressure signals are indicative of a pressure in the wearable cuff 14 during a respective period of a plurality of periods for measuring blood pressure. The plurality of pressure signals may thus have been obtained for a particular wearable cuff 14. A period for measuring blood pressure can be any period, or any combination of periods, in which blood pressure can be measured. For example, in some embodiments, the period for measuring blood pressure may comprise a period in which the wearable cuff 14 is inflated and/or deflated for measuring blood pressure. For example, the period for measuring blood pressure may comprise a period for inflation-based NIBP measurement and/or deflation-based NIBP measurement. In some embodiments, the periods for measuring blood pressure can be measurement instances. That is, the periods for measuring blood pressure can be periods in which blood pressure is measured according to some embodiments. Thus, in some embodiments, each of the plurality of pressure signals can be indicative of a pressure in the wearable cuff 14 over the course of a blood pressure measurement. In some embodiments, the periods for measuring blood pressure may comprise periods for measuring blood pressure that occur within a predefined time period preceding the acquisition (e.g. within a recent time period) of the plurality of pressure signals.

At block 204 of Fig. 2, for each of the plurality of acquired pressure signals, a measure of a quality of the acquired pressure signal is determined based on one or more parameters derived from artifacts identified (or detected) in the acquired pressure signal. More specifically, for each of the plurality of acquired pressure signals, the apparatus 12 determines the measure of the quality of the acquired pressure signal based on one or more parameters derived from artifacts identified in the acquired pressure signal. Thus, in effect, a quality assessment of the acquired plurality of pressure signals is made based upon the identified artifacts. The artifacts referred to herein may comprise artifacts due to cuff slippage and/or any other artifacts.

As each of the plurality of pressure signals are indicative of a pressure in the wearable cuff 14 during a period for measuring blood pressure, the one or more parameters can be derived from artifacts identified in the acquired pressure signal during the periods for measuring blood pressure. For example, in some embodiments, the one or more parameters can be derived from artifacts identified in the acquired pressure signal during one or more periods in which the wearable cuff 14 is inflated for measuring blood pressure (e.g. one or more periods of inflation-based NIBP measurement) and/or one or more periods in which the wearable cuff 14 is deflated for measuring blood pressure (e.g. one or more periods of deflation-based NIBP measurement). As mentioned earlier, in some embodiments, the periods for measuring blood pressure can be measurement instances. That is, the periods for measuring blood pressure can be periods in which blood pressure is measured according to some embodiments. Thus, in some embodiments, the one or more parameters can be derived from artifacts identified in the acquired pressure signal over the course of a blood pressure measurement.

In this way, measures of a quality of the plurality of acquired pressure signals can be determined. As the plurality of acquired pressure signals are each indicative of a pressure in the wearable cuff 14 during a period for measuring blood pressure, the plurality of acquired pressure signals can each be indicative of a pressure in the wearable cuff 14 during a different period for measuring blood pressure. Thus, in effect, a time series of measures of the quality of the plurality of acquired pressure signals can be determined. In embodiments where the plurality of acquired pressure signals comprise one or more historical pressure signals, for example, a historical time series of measures of the quality of the plurality of acquired pressure signals can be determined. The measures of a quality of the acquired pressure signals determined in the manner described herein can allow the quality of the acquired pressure signals to be tracked over time. Thus, in some embodiments, the apparatus 12 can be configured to track the determined measures of the quality of the acquired pressure signals over time.

In some embodiments, the apparatus 12 may be configured to determine the measures of the quality of the acquired pressure signals based on one or more parameters derived from artifacts identified in the acquired pressure signal using a machine learning algorithm. For example, in some embodiments, the apparatus 12 may be configured to determine the measures of the quality of the acquired pressure signals using any one or more of a trained classifier, a regression analysis, a support vector machine (SVM), and a neural network algorithm. Although examples have been provided for a machine learning algorithm that may be used, it will be understood that other machine learning algorithms may also be used and a person skilled in the art will be aware of such machine learning algorithms.

A machine learning algorithm can learn a target function that best maps an input to an output. Once a target function is learnt, the machine learning algorithm can then provide an output given any new input. For example, a machine learning algorithm may operate by building a model from an example training set of inputs (e.g. obtained from a plurality of wearable cuffs having a known measure of quality, such as at least one high quality wearable cuff and at least one low quality wearable cuff) in order to make data-driven predictions or decisions as outputs. Here, the inputs to the machine learning algorithm are the one or more parameters derived from artifacts identified in each of the acquired pressure signal and the outputs of the machine learning algorithm are the measures of the quality of the acquired pressure signals. That is, the outputs of the machine learning algorithm can be decisions on the quality of the acquired pressure signals.

The measures of the quality of the acquired pressure signals referred to herein can, for example, comprise binary measures of the quality of the acquired pressure signal, probabilistic measures of the quality of the acquired pressure signals, or any other suitable measures of the quality of the acquired pressure signals. In an example embodiment involving binary measures of the quality of the acquired pressure signal, the measure of the quality of the acquired pressure signal may be labelled as 0 or 1. For example, a label of 0 may be representative of a low quality pressure signal and a label of 1 may be representative of a high quality pressure signal or vice versa.

In an example embodiment involving probabilistic measures of the quality of the acquired pressure signal, the measure of the quality of the acquired pressure signal may be labelled as a fraction (or ratio), a percentage, or any other probabilistic measure. The probabilistic measures of the quality of the acquired pressure signal can be indicative of a position of the acquired pressure signals on a quality scale (e.g. from 0-1 or 0-100%, etc.). For example, the bottom end of the scale can be indicative of a low quality pressure signal and the top end of the scale can be indicative of a high quality pressure signal or vice versa.

The one or more parameters referred to herein that are derived from artifacts identified in the acquired pressure signal may be any suitable parameter, or any combination of suitable parameters, on which a determination of a measure of a quality of an acquired pressure signal can be based. For example, the one or more parameters derived from artifacts identified in the acquired pressure signal may comprise a number of artifacts (e.g. a number of occurrences of artifacts) in the acquired pressure signal. In some embodiments, for example, a number of artifacts (e.g. a number of occurrences of artifacts) in the acquired pressure signal above a maximum threshold may be deemed too many. Alternatively or in addition, the one or more parameters derived from artifacts identified in the acquired pressure signal may comprise a number of artifacts (e.g. a number of occurrences of artifacts) in the acquired pressure signal that fall within a cardiac cycle. The cardiac cycle may be defined as a time period between diastole and systole.

Alternatively or in addition, the one or more parameters derived from artifacts identified in the acquired pressure signal may comprise an average time or duration between artifacts (e.g. occurrences of artifacts) in the acquired pressure signal. That is, the one or more parameters derived from artifacts identified in the acquired pressure signal may comprise an average frequency of artifacts (e.g. occurrences of artifacts) in the acquired pressure signal. The average frequency of artifacts (e.g. occurrences of artifacts) in the acquired pressure signal can, for example, comprise the average number of artifacts (e.g. occurrences of artifacts) in the acquired pressure signal per unit time (e.g. per second) or per unit pressure change in the wearable cuff 14. In some embodiments, for example, an average frequency of artifacts (e.g. occurrences of artifacts) in the acquired pressure signal may be deemed too frequent. Alternatively or in addition, the one or more parameters derived from artifacts identified in the acquired pressure signal may comprise an average intensity of artifacts in the acquired pressure signal. For example, the one or more parameters derived from artifacts identified in the acquired pressure signal may comprise a number of artifacts in the acquired pressure signal with an intensity above a predetermined threshold. In some embodiments, for example, artifacts in the acquired pressure signal with an intensity above the predetermined threshold may be deemed too severe.

Alternatively or in addition, the one or more parameters derived from artifacts identified in the acquired pressure signal may comprise a ratio of a cumulative sum of amplitudes of artifacts in the acquired pressure signal to a cumulative sum of amplitudes of oscillations (e.g. pressure oscillations) in the acquired pressure signal. Alternatively or in addition, the one or more parameters derived from artifacts identified in the acquired pressure signal may comprise a ratio of an average of amplitudes of artifacts in the acquired pressure signal to an average of amplitudes of oscillations (e.g. pressure oscillations) in the acquired pressure signal. Alternatively or in addition, the one or more parameters derived from artifacts identified in the acquired pressure signal may comprise a ratio of a maximum of amplitudes of artifacts in the acquired pressure signal to a maximum of amplitudes of oscillations (e.g. pressure oscillations) in the acquired pressure signal.

Although some examples have been provided for the one or more parameters derived from artifacts identified in the acquired pressure signal, it will be understood that other parameters and any combinations of parameters are possible.

Returning back to Fig. 2, at block 206, a measure of a quality of the wearable cuff 14 is determined based on the determined measures of the quality of the acquired pressure signals. More specifically, the apparatus 12 determines the measure of the quality of the wearable cuff 14 based on the determined measures of the quality of the acquired pressure signals. In some embodiments, the apparatus 12 may be configured to determine the measure of a quality of the wearable cuff 14 based on the determined measures of the quality of the acquired pressure signals by being configured to determine the measure of a quality of the wearable cuff 14 based on one or more features of the determined measures of the quality of the acquired pressure signals over time. The one or more features of the determined measures of the quality of the acquired pressure signals over time may, for example, comprise any one or more of a slope of the measures of the quality of the acquired pressure signal over time, a weighted average of the measures of a quality of the wearable cuff 14 over time, and an acceleration (or a deceleration) in the measures of a quality of the wearable cuff 14 over time.

Since one or more of the measures of the quality can be of an acquired pressure signal that is current (e.g. a pressure signal acquired from at least one pressure sensor) and/or an acquired pressure signal that is historical (e.g. a pressure signal acquired from at least one memory), the measure of the quality of the wearable cuff 14 can be based on one more current and/or historical measures of the quality of the acquired pressure signals according to some embodiments. Thus, in some embodiments, an overall assessment of the quality of the wearable cuff 14 can be made based on the current quality and/or the history of the quality of the pressure signals acquired for the wearable cuff 14. As mentioned earlier, the determined measures of the quality of the acquired pressure signals can be a time series (e.g. a historical time series) of measures of the quality of the plurality of acquired pressure signals. Thus, in this way, an overall assessment of the quality of the wearable cuff 14 can be made based on a time series (e.g. a historical time series) of measures of the quality of the plurality of acquired pressure signals.

As the overall assessment of the quality of the wearable cuff described herein is made based on measures of a quality of a plurality of acquired pressure signal in respect of various periods for measuring blood pressure and using one or more parameters derived from artifacts identified in the plurality of acquired pressure signal, the quality assessment made by way of the technique described herein is more reliable since confounding factors (e.g. cuff slippage) that can affect the quality of pressure signals for a wearable cuff 14 can be detected and accounted for in the measures of the quality of the acquired pressure signals that are determined. For example, the technique described herein can identify situations where confounding factors (e.g. cuff slippage) are affecting the quality of pressure signals for a wearable cuff 14, while the wearable cuff 14 itself is actually of a high quality, and can thus provide a more reliable quality assessment for the wearable cuff 14.

In some embodiments, the apparatus 12 may be configured to determine the measure of the quality of the wearable cuff 14 based on a trend (e.g. a historical trend) in the determined measures of the quality of the acquired pressure signals. As there may be confounding factors (e.g. cuff slippage) that can affect the quality of pressure signals for a wearable cuff 14, while the wearable cuff 14 itself may be of a high quality, an even more reliable assessment of the quality of the wearable cuff 14 can be obtained by analyzing the trend (e.g. a historical trend) in the determined measures of the quality of the acquired pressure signals in order to determine a measure of the quality of the wearable cuff 14.

In some embodiments, the apparatus 12 can be configured to, for each of the plurality of acquired pressure signals, determine the measure of the quality of the acquired pressure signal by being configured to compare the one or more parameters to one or more corresponding parameters associated with at least one wearable cuff 14 for which a measure of a quality of the at least one wearable cuff 14 is predetermined.

In some embodiments, the apparatus 12 can be configured to determine the measure of the quality of the wearable cuff 14 based on the determined measures of the quality of the acquired pressure signals and also information on one or more properties of the wearable cuff 14. In some embodiments, the apparatus 12 can be configured to determine the measure of the quality of the wearable cuff 14 based on the determined measures of the quality of the acquired pressure signals and the information on one or more properties of the wearable cuff 14 using a machine learning algorithm (such as any of those mentioned earlier). In some embodiments, a common machine learning algorithm may be used for both the determined measures of the quality of the acquired pressure signals and the information on one or more properties of the wearable cuff 14. In other embodiments, a separate machine learning algorithm may be used for the determined measures of the quality of the acquired pressure signals and the information on one or more properties of the wearable cuff 14.

The information on one or more properties of the wearable cuff 14 can, for example, comprise information on a build and/or size of the wearable cuff 14 (e.g. a cuff type) and/or a make of the wearable cuff 14 (e.g. a manufacturing code or tracking number). In some embodiments, the apparatus 12 can be configured to acquire or access the information on one or more properties of the wearable cuff 14 from at least one memory. In some embodiments, the information on one or more properties of the wearable cuff 14 may be uploaded electronically on the wearable cuff 14, such that the apparatus 12 can acquire or access the information from the wearable cuff 14. Taking into account one or more properties of the wearable cuff 14 in the quality assessment for the wearable cuff 14 can further improve the accuracy and reliability of the quality assessment since the quality (and also the lifetime) of the wearable cuff 14 can depend on such properties.

In some embodiments, in the same way that the apparatus 12 may be configured to determine the measures of the quality of the acquired pressure signals based on one or more parameters derived from artifacts identified in the acquired pressure signal using a machine learning algorithm, the apparatus 12 may be configured to determine the measure of the quality of the wearable cuff 14 based on the determined measures of the quality of the acquired pressure signals and optionally also information on one or more properties of the wearable cuff 14 using a machine learning algorithm (such as any of those described earlier).

As mentioned earlier a machine learning algorithm may operate by building a model from an example training set of inputs (e.g. obtained from a plurality of wearable cuffs having a known measure of quality, such as at least one high quality wearable cuff and at least one low quality wearable cuff) in order to make data-driven predictions or decisions as outputs. Here, the inputs to the machine learning algorithm are the determined measures of the quality of the acquired pressure signals (and/or the one or more features of the determined measures of the quality of the acquired pressure signals over time mentioned earlier according to some embodiments) and the output of the machine learning algorithm is the measure of the quality of the wearable cuff 14. That is, the output of the machine learning algorithm can be a decision on the quality of the wearable cuff 14.

The measure of the quality of the wearable cuff 14 referred to herein can, for example, comprise a binary measure of the quality of the wearable cuff 14, probabilistic measures of the quality of the wearable cuff 14, or any other suitable measure of the quality of the wearable cuff 14. In an example embodiment involving binary measures of the quality of the wearable cuff 14, the measure of the quality of the wearable cuff 14 may be labelled as 0 or 1. For example, a label of 0 may be representative of a low quality wearable cuff and a label of 1 may be representative of a high quality wearable cuff or vice versa.

In an example embodiment involving probabilistic measures of the quality of the wearable cuff 14, the measure of the quality of the wearable cuff 14 may be labelled as a fraction (or ratio), a percentage, or any other probabilistic measure. The probabilistic measures of the quality of the wearable cuff 14 can be indicative of a position of the wearable cuff 14 on a quality scale (e.g. from 0-1 or 0-100%, etc.). For example, the bottom end of the scale can be indicative of a low quality wearable cuff and the top end of the scale can be indicative of a high quality wearable cuff or vice versa. In this way, it is possible to provide inference on a magnitude of the quality of the wearable cuff 14.

In some embodiments, the wearable cuff 14 can be classified into a category representative of a particular quality, e.g. a low quality category or a high quality category. In some embodiments involving binary measures, the wearable cuff 14 may be classified into a low quality category when all of the measures of the quality of the acquired pressure signals are classified into a low quality category. Thus, in some embodiments, the determined measure of the quality of the wearable cuff 14 can be indicative of whether the wearable cuff 14 is classified as a high quality wearable cuff 14 (e.g. a wearable cuff 14 that is capable of providing high quality pressure signals or pressure signals) or a low quality wearable cuff 14 (e.g. a wearable cuff 14 that is capable of providing only low quality pressure signals or pressure signals).

In some embodiments, the determined measure of the quality of the wearable cuff 14 can be indicative of a suitability of the wearable cuff 14 for use in measuring blood pressure. For example, in some embodiments, the determined measure of the quality of the wearable cuff 14 may be indicative of the wearable cuff 14 being suitable (or unsuitable) for use in measuring blood pressure during inflation of the wearable cuff 14. Alternatively or in addition, in some embodiments, the determined measure of the quality of the wearable cuff 14 may be indicative of the wearable cuff 14 being suitable (or unsuitable) for use in measuring blood pressure during deflation of the wearable cuff 14. Alternatively, the determined measure of the quality of the wearable cuff 14 may be indicative of the wearable cuff 14 being unsuitable for use in measuring blood pressure (e.g. unsuitable for use in both inflation-based and deflation based blood pressure measurement). In some embodiments, the wearable cuff 14 may be provided with an indication (e.g. marked, such as via color coding or printed stickers) of its suitability for use in measuring blood pressure.

In some embodiments, the determined measure of the quality of the wearable cuff 14 can be indicative of the wearable cuff 14 being unsuitable for use in measuring blood pressure when the determined measure of the quality of the wearable cuff 14 is below a predefined threshold. In some embodiments where the determined measure of the quality of the wearable cuff 14 is indicative of the wearable cuff 14 being unsuitable for use in measuring blood pressure, the apparatus 12 may be configured to skip or abort (or control a blood pressure measurement apparatus to skip or abort) a blood pressure measurement.

In some embodiments where the determined measure of the quality of the wearable cuff 14 is indicative of the wearable cuff 14 being unsuitable for use in measuring blood pressure during inflation of the wearable cuff 14, the apparatus 12 may be configured to skip or abort (or control a blood pressure measurement apparatus to skip or abort) the inflation-based blood pressure measurement and to instead proceed with measuring blood pressure during deflation of the wearable cuff 14. A pressure signal acquired during deflation of the wearable cuff 14 is generally less affected by artifacts than a pressure signal acquired during inflation of the wearable cuff 14 and can thus a blood pressure measurement determined from such a signal can have a higher quality. In an example, a wearable cuff 14 may be in use during an inflation-based NIBP measurement and, during that measurement, a low cuff quality may be determined. In some embodiments, this may cause the inflation-based NIBP measurement to stop (e.g. immediately) and optionally this may be proceeded (e.g. immediately) by a deflation-based NIBP measurement.

Although not illustrated in Fig. 2, in some embodiments, the method 200 may comprise determining whether the wearable cuff 14 is to be discarded or replaced based on the determined measure of the quality of the wearable cuff 14. That is, in some embodiments, the apparatus 12 can be configured to determine whether the wearable cuff 14 is to be discarded or replaced based on the determined measure of the quality of the wearable cuff 14. In some embodiments, where it is determined that the wearable cuff 14 is to be discarded or replaced, the method 200 may comprise controlling a user interface (such as that described earlier) to render an indication that the wearable cuff 14 is to be discarded or replaced. That is, in some embodiments, the apparatus 12 can be configured to control the user interface to render the indication of that the wearable cuff 14 is to be discarded or replaced. In this way, a user can be made aware of when to discard or replace a wearable cuff 14.

Although also not illustrated in Fig. 2, in some embodiments, the method 200 may comprise identifying the wearable cuff 14 for which the measure of the quality is determined. That is, in some embodiments, the apparatus 12 can be configured to identify the wearable cuff 14 for which the measure of the quality is determined. This may be the case, for example, where the apparatus 12 is used with (e.g. connected to) different wearable cuffs 14. In some embodiments, the wearable cuff 14 may comprise an identifier that enables the identification of the wearable cuff 14. For example, in some embodiments, the identifier may be a marker, such as a barcode, a color coding, a sticker (e.g. with the identity of the wearable cuff 14 printed on it), or any other identifier, or any combination of identifiers. In some embodiments, the identifier may be detected by the apparatus 12, e.g. via a camera, a barcode reader, or similar. Alternatively or in addition, in some embodiments, the wearable cuff 14 may comprise a radio frequency identification (RFID) chip that is readable by the apparatus 12 for the apparatus 12 to identify the wearable cuff 14. In some embodiments, a memory (such as that described earlier) may store identities of wearable cuffs 14 and measures of the quality of the wearable cuffs 14 (e.g. in a look-up table) for retrieval by the apparatus 12.

In some embodiments, for each wearable cuff 14, the measure of the quality of the wearable cuff 14 determined per period for measuring blood pressure may be stored in a memory (such as that described earlier). For example, the apparatus 12 itself may be configured to store the measures of the quality of the wearable cuffs 14 or the measures of the quality of the wearable cuff 14 may be stored at another location, such as a network location that can communicate (e.g. wirelessly) with a plurality of apparatus 12. In this case, measures of the quality of wearable cuffs 14 determined by multiple apparatus 12 may be combined (e.g. pooled together) to provide an improved assessment of cuff quality.

In other embodiments, the identity of the wearable cuff 14 for which the measure of the quality is determined may already be known. Thus, identification of the wearable cuff 14 may not be required. For example, this may be the case where the apparatus 12 is always used with (e.g. connected to) the same wearable cuff 14. In some of these embodiments, the apparatus 12 may have access to a history of measures of the quality of the wearable cuff 14 and may use that history in determining the measure of the quality of the wearable cuff 14 based on one more current and historical measures of the quality of the acquired pressure signals according to some embodiments. It can also be the case that identification of the wearable cuff 14 may not be required where the apparatus 12 is used with (e.g. connected to) the same wearable cuff 14 for a time period that extends beyond a predefined threshold (e.g. a long time period that lasts several hours or days, such as when the apparatus 12 is in an automated spot-check mode). In these embodiments, the apparatus 12 may use historical measures of the quality of the acquired pressure signals for the period that the apparatus 12 is connected to the same wearable cuff 14 and the current measure of the quality of the acquired pressure signals to determine the measure of the quality of the wearable cuff 14.

Although also not illustrated in Fig. 2, in some embodiments, the method 200 may comprise controlling a user interface (such as that described earlier) to render an indication of the measure of the quality of the wearable cuff 14. That is, in some embodiments, the apparatus 12 can be configured to control the user interface to render the indication of the measure of the quality of the wearable cuff 14.

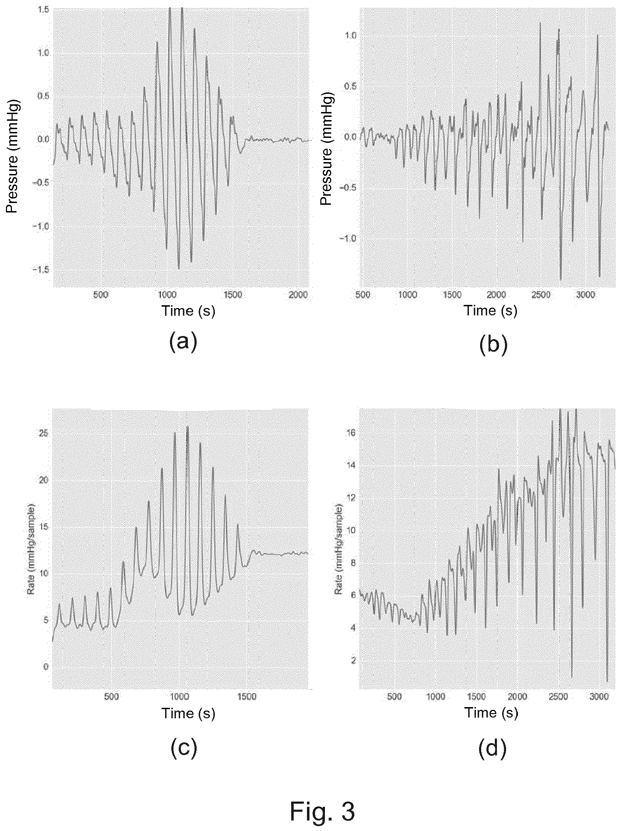

Figs. 3(a) and (b) are each a graphical illustration of a pressure signal indicative of a pressure in a wearable cuff 14 (measured in mmHg) over time (measured in seconds) during a period for measuring blood pressure for two different wearable cuffs 14 according to an embodiment. The pressure signals include oscillations in pressure. In more detail, Fig. 3(a) is a graphical illustration of a pressure signal for a wearable cuff 14 that is classified as a high quality wearable cuff according to existing techniques and Fig. 3(b) is a graphical illustration of a pressure signal for a wearable cuff 14 that is classified as a low quality wearable cuff according to existing techniques. As can be seen in Fig. 3(b), the pressure signal for the wearable cuff 14 classified as a low quality wearable cuff according to existing techniques is in fact corrupted by external factors (e.g. cuff slippage) and this can lead to an erroneous assessment of the quality of the wearable cuff 14 using existing techniques.

Figs. 3(c) and (d) are each a graphical illustration of a rate signal over time during the period for measuring blood pressure for the two different wearable cuffs according to the embodiment. More specifically, Fig. 3(c) is a graphical illustration of a rate signal for a wearable cuff 14 that is classified as a high quality wearable cuff according to existing techniques and Fig. 3(d) is a graphical illustration of a rate signal for a wearable cuff 14 that is classified as a low quality wearable cuff according to existing techniques. From the rate signals illustrated in Fig. 3(c) and (d), it is possible to better visualize the occurrences of confounding factors (e.g. cuff slippage) affecting the quality of pressure signals for a wearable cuff 14. As illustrated in Fig. 3(d), the rate signal for the wearable cuff 14 that is classified as a low quality wearable cuff exhibits various sharp drops over time, which are due to confounding factors (e.g. cuff slippage). As illustrated in Fig. 3(c), these sharp drops are not seen in the rate signal for the wearable cuff 14 that is classified as a high quality wearable cuff.

By way of the apparatus and method described herein, it is possible to identify situations where confounding factors (e.g. cuff slippage) are affecting the quality of pressure signals for a wearable cuff 14, while the wearable cuff 14 itself is actually of a high quality, since the overall assessment of the quality of the wearable cuff described herein is made based on measures of a quality of a plurality of acquired pressure signal in respect of various periods for measuring blood pressure and using one or more parameters derived from artifacts identified in the plurality of acquired pressure signal. In this way, a more reliable quality assessment for the wearable cuff 14 is provided.

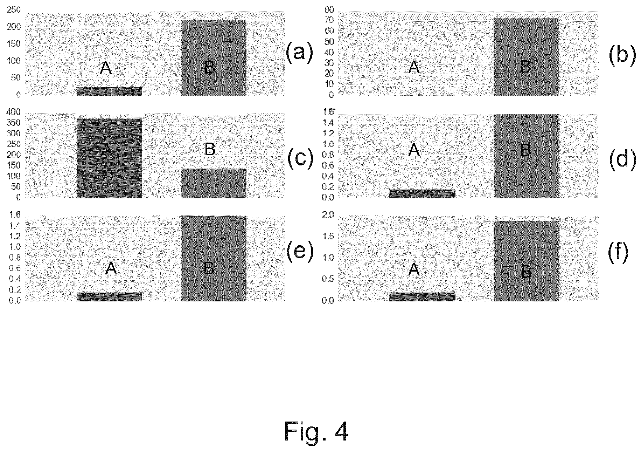

Figs. 4(a)-(f) are graphical illustrations of example parameters derived from artifacts identified in an acquired pressure signal from two different cuffs according to the embodiment. In more detail, Fig. 4(a) is a graphical illustration of the parameter of the number of occurrences of artifacts in the acquired pressure signal for a wearable cuff A classified as a high quality wearable cuff and for a wearable cuff B classified as a low quality wearable cuff. Fig. 4(b) is a graphical illustration of the parameter of the number of occurrences of artifacts in the acquired pressure signal that fall within a cardiac cycle for the wearable cuff A classified as a high quality wearable cuff and for the wearable cuff B classified as a low quality wearable cuff. Fig. 4(c) is a graphical illustration of the parameter of the average time between occurrences of artifacts in the acquired pressure signal for the wearable cuff A classified as a high quality wearable cuff and for the wearable cuff B classified as a low quality wearable cuff.

Fig. 4(d) is a graphical illustration of the parameter of the ratio of a cumulative sum of amplitudes of artifacts in the acquired pressure signal to a cumulative sum of amplitudes of oscillations in the acquired pressure signal for the wearable cuff A classified as a high quality wearable cuff and for the wearable cuff B classified as a low quality wearable cuff. Fig. 4(e) is a graphical illustration of the parameter of the ratio of an average of amplitudes of artifacts in the acquired pressure signal to an average of amplitudes of oscillations in the acquired pressure signal for the wearable cuff A classified as a high quality wearable cuff and for the wearable cuff B classified as a low quality wearable cuff. Fig. 4(f) is a graphical illustration of the parameter of the ratio of a maximum of amplitudes of artifacts in the acquired pressure signal to a maximum of amplitudes of oscillations in the acquired pressure signal for the wearable cuff A classified as a high quality wearable cuff and for the wearable cuff B classified as a low quality wearable cuff.

Thus, as illustrated in Figs. 4(a)-(f), for each of the parameters, there are differences between (and, in fact, a significant separation between) the parameter depending on the quality of the wearable cuff 14. Therefore, it can be seen from Figs. 4(a)-(f) that the one or more parameters derived from artifacts identified in an acquired pressure signal can provide a feasible and reliable measure of the quality of a wearable cuff 14 in the manner described herein.

In some embodiments, the measure of the quality of a wearable cuff 14 may be determined during a clinical monitoring workflow. For example, the plurality of pressure signals used in the determination may be acquired as part of clinical monitoring of a subject. In these embodiments, the plurality of pressure signals may be acquired while the wearable cuff 14 is worn on or around (e.g. wrapped around, attached to, or fastened to) a measurement site of the subject.

In other embodiments, the measure of the quality of a wearable cuff 14 may be determined separately to such clinical monitoring. For example, in some embodiments, the measure of the quality of a wearable cuff 14 may be determined in a service (or test) mode that is dedicated to cuff quality assessment. Thus, in some embodiments, the cuff quality assessment can be performed outside a medical monitoring period. A service (or test) mode dedicated to cuff quality assessment can allow a standardized procedure to be introduced for cuff quality assessment that limits the influence of subjects and other users (e.g. nurses, doctors, or other medical personnel). Therefore, in some embodiments, a reliable measure of the quality of a wearable cuff 14 may be determined based on a single measurement instance.

In some of these embodiments, the plurality of pressure signals may be acquired while the wearable cuff 14 is worn on or around (e.g. wrapped around, attached to, or fastened to) a phantom, e.g. an arm phantom. In some embodiments, the phantom may comprise no active elements. For example, in some embodiments, the phantom may be a rigid cylinder. The phantom may have a fixed geometry or a geometry that can be adjusted to different size wearable cuffs 14. In some embodiments, the phantom may comprise active elements, e.g. to improve the procedure. For example, in some embodiments, the phantom may comprise active elements (e.g. one or more pressure sensors inside the phantom) configured to characterize a wrapping of the wearable cuff 14 and/or to provide guidance in the positioning of the wearable cuff 14, e.g. taking into account the application of the wearable cuff 14. An example service (or test) mode procedure may consist of wrapping a wearable cuff 14 around a phantom according to a standard procedure and initiating a blood pressure measurement. The plurality of pressure signals may then be acquired, where each of the plurality of pressure signals are indicative of a pressure in the wearable cuff 14 during a period for measuring blood pressure. Thus, for example, the procedure can consist of one or more sequences of inflation-based and/or deflation-based blood pressure measurements, e.g. at different inflation and/or deflation speeds and/or maximum pressures. The measure of the quality of the wearable cuff 14 can be determined in the manner described earlier.

In some embodiments, the measure of the quality of a wearable cuff 14 determined in the manner described herein can be used to identify wearable cuffs 14 that are appropriate for blood pressure measurements and wearable cuffs 14 that are inappropriate for blood pressure measurements. In this way, the burden on the subject or other users of the apparatus 12 (e.g. nurses, doctors, or other medical personnel) can be eased as they can easily and reliably identify appropriate and inappropriate wearable cuffs 14. In some embodiments, blood pressure measurements may be limited to a particular wearable cuff 14 based on measures of the quality of a plurality of wearable cuffs 14 determined in the manner described herein. In some embodiments, the apparatus 12 may be configured to (e.g. automatically) limit blood pressure measurements in this way.

In some embodiments, the apparatus 12 may be configured to (e.g. automatically) keep track of wearable cuffs 14 in terms of the measure of the quality of the wearable cuffs 14 determined in the manner described herein. In some embodiments, the apparatus 12 may be configured to output recommendations (e.g. via a user interface such as that described earlier) based on measures of the quality of the wearable cuffs 14 determined in the manner described herein. For example, the recommendation may comprise an indication of the manner in which the measure of the quality of a wearable cuff 14 can be improved (e.g. by cleaning the wearable cuff 14). The measure of the quality of a wearable cuff 14 determined in the manner described herein can be useful in improving logistics in medical environments (e.g. hospitals), such as by providing the information required to manually or automatically order new wearable cuffs 14 at appropriate times. The measure of the quality of a wearable cuff 14 determined in the manner described herein may also be useful in training sessions, e.g. by providing feedback on wrapping styles in order to aid in improving the application of a wearable cuff 14 to a subject.

In some embodiments, any one or more of the steps that the apparatus 12 described herein is configured to perform may be performed during a blood pressure measurement. Alternatively or in addition, in some embodiments, at least one or all of the steps that the apparatus 12 is configured to perform can be automated.

There is also described a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an apparatus 12, a system 10, a method 200 and a computer program product that address the limitation associated with the existing techniques. Therefore, there is provided an improved technique for assessing the quality of a wearable cuff 14 for use in measuring blood pressure.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (12) for use with a wearable cuff (14), wherein the wearable cuff (14) is for use in measuring blood pressure and the apparatus (12) is configured to:
acquire a plurality of pressure signals, wherein each of the plurality of pressure signals are indicative of a pressure in the wearable cuff (14) during a period for measuring blood pressure;
for each of the plurality of acquired pressure signals, determine a measure of a quality of the acquired pressure signal based on one or more parameters derived from artifacts identified in the acquired pressure signal; and
determine a measure of a quality of the wearable cuff (14) based on the determined measures of the quality of the acquired pressure signals.

2. The apparatus (12) as claimed in claim 1, wherein the apparatus (12) is configured to:
determine the measure of the quality of the wearable cuff (14) based on a trend in the determined measures of the quality of the acquired pressure signals.

3. The apparatus (12) as claimed in any preceding claim, wherein the apparatus (12) is configured to:
determine the measure of the quality of the wearable cuff (14) based on the determined measures of the quality of the acquired pressure signals and information on one or more properties of the wearable cuff (14).

4. The apparatus (12) as claimed in any of the preceding claims, wherein the apparatus (12) is configured to:
for each of the plurality of acquired pressure signals, determine the measure of the quality of the acquired pressure signal by being configured to:
compare the one or more parameters to one or more corresponding parameters associated with at least one wearable cuff (14) for which a measure of a quality of the at least one wearable cuff (14) is predetermined.

5. The apparatus (12) as claimed in any of the preceding claims, wherein the measures of the quality of the acquired pressure signals comprise:
binary measures of the quality of the acquired pressure signals; or
probabilistic measures of the quality of the acquired pressure signals.

6. The apparatus (12) as claimed in any of the preceding claims, wherein the determined measure of the quality of the wearable cuff (14) is indicative of a suitability of the wearable cuff (14) for use in measuring blood pressure.

7. The apparatus (12) as claimed in claim 6, wherein the determined measure of the quality of the wearable cuff (14) is indicative of:
the wearable cuff (14) being suitable for use in measuring blood pressure during inflation of the wearable cuff (14);
the wearable cuff (14) being suitable for use in measuring blood pressure during deflation of the wearable cuff (14); or
the wearable cuff (14) being unsuitable for use in measuring blood pressure.

8. The apparatus (12) as claimed in any of the preceding claims, wherein the apparatus (12) is configured to:
determine whether the wearable cuff (14) is to be discarded or replaced based on the determined measure of the quality of the wearable cuff (14).

9. The apparatus (12) as claimed in any of the preceding claims, wherein the one or more parameters comprise any one or more of:
a number of occurrences of artifacts in the acquired pressure signal;
a number of occurrences of artifacts in the acquired pressure signal that fall within a cardiac cycle;
an average time between occurrences of artifacts in the acquired pressure signal;
an average intensity of artifacts in the acquired pressure signal;
a ratio of a cumulative sum of amplitudes of artifacts in the acquired pressure signal to a cumulative sum of amplitudes of oscillations in the acquired pressure signal;
a ratio of an average of amplitudes of artifacts in the acquired pressure signal to an average of amplitudes of oscillations in the acquired pressure signal; and
a ratio of a maximum of amplitudes of artifacts in the acquired pressure signal to a maximum of amplitudes of oscillations in the acquired pressure signal.

10. The apparatus (12) as claimed in any of the preceding claims, wherein the period for measuring blood pressure comprises:
a period in which the wearable cuff (14) is inflated and/or deflated for measuring blood pressure.

11. The apparatus (12) as claimed in any of the preceding claims, wherein the apparatus (12) is configured to determine the measures of the quality of the acquired pressure signals using any one or more of:
a trained classifier;
a regression analysis;
a support vector machine; and
a neural network algorithm.

12. The apparatus (12) as claimed in any of the preceding claims, wherein the apparatus (12) is configured to:
identify the wearable cuff (14) for which the measure of the quality is determined.

13. The apparatus (12) as claimed in any of the preceding claims, wherein the apparatus (12) is configured to:
control a user interface to render an indication of the measure of the quality of the wearable cuff (14).

14. A method (200) of operating an apparatus (12) for use with a wearable cuff (14), wherein the wearable cuff (14) is for use in measuring blood pressure and the method (200) comprises:
acquiring (202) a plurality of pressure signals, wherein each of the plurality of pressure signals are indicative of a pressure in the wearable cuff (14) during a period for measuring blood pressure;
for each of the plurality of acquired pressure signals, determining (204) a measure of a quality of the acquired pressure signal based on one or more parameters derived from artifacts identified in the acquired pressure signal; and
determining (206) a measure of a quality of the wearable cuff (14) based on the determined measures of the quality of the acquired pressure signals.

15. A computer program product comprising a computer readable medium, the computer readable medium having computer readable code embodied therein, the computer readable code being configured such that, on execution by a suitable computer or processor, the computer or processor is caused to perform the method as claimed in claim 14.
